# EUROPEAN PATENT SPECIFICATION

(11) **EP 2 399 646 B1**
(45) Date of publication and mention of the grant of the patent: **18.09.2013**
(21) Application number: 11004942.6
(22) Date of filing: 16.06.2011
(51) Int. Cl.: A61N 5/04, A61B 18/18, A61B 18/00, A61B 18/14

(54) **Microwave ground plane antenna probe**
Mikrowellen-Groundplane-Antennensonde
Sonde d'antenne à plan de base à micro-ondes

(30) Priority: 25.06.2010 US 823211
(43) Date of publication of application: 28.12.2011
(73) Proprietor: Covidien LP, Mansfield, MA 02048 (US)
(72) Inventor: Prakash, Mani N., Boulder CO 80301 (US); Rossetto, Francesca, Longmont CO 80504 (US); Brannan, Joseph D., Erie CO 80516 (US); Nguyen, Tao, Redwood City CA 94063 (US)
(74) Representative: Beyer, Andreas

(56) References cited:
- EP-A1- 2 174 614
- WO-A1-2006/084676
- WO-A2-02/061880
- US-A1- 2003 100 894
- US-A1- 2007 185 554

## Description

### BACKGROUND

### 1. Technical Field

The present disclosure relates to systems for providing energy to biological tissue and, more particularly, to a microwave ablation surgical probe having a conductive ground plane.

### 2. Background of Related Art

Energy-based tissue treatment is well known in the art. Various types of energy (e.g., electrical, ultrasonic, microwave, cryogenic, thermal, laser, etc.) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator. In tissue ablation electrosurgery, the radio frequency energy may be delivered to targeted tissue by an antenna or probe.

There are several types of microwave antenna assemblies in use, e.g., monopole, dipole and helical, which may be used in tissue ablation applications. In monopole and dipole antenna assemblies, microwave energy generally radiates perpendicularly away from the axis of the conductor. Monopole antenna assemblies typically include a single, elongated conductor. A typical dipole antenna assembly includes two elongated conductors, which are linearly aligned and positioned end-to-end relative to one another with an electrical insulator placed therebetween. Helical antenna assemblies include a helically-shaped conductor connected to a ground plane. Helical antenna assemblies can operate in a number of modes including normal mode (broadside), in which the field radiated by the helix is maximum in a perpendicular plane to the helix axis, and axial mode (end fire), in which maximum radiation is along the helix axis. The tuning of a helical antenna assembly may be determined, at least in part, by the physical characteristics of the helical antenna element, e.g., the helix diameter, the pitch or distance between coils of the helix, and the position of the helix in relation to the probe assembly to which it is mounted.

The typical microwave antenna has a long, thin inner conductor that extends along the longitudinal axis of the probe and is surrounded by a dielectric material and is further surrounded by an outer conductor around the dielectric material such that the outer conductor also extends along the axis of the probe. In another variation of the probe that provides for effective outward radiation of energy or heating, a portion or portions of the outer conductor can be selectively removed. This type of construction is typically referred to as a "leaky waveguide" or "leaky coaxial" antenna. Another variation on the microwave probe involves having the tip formed in a uniform spiral pattern, such as a helix, to provide the necessary configuration for effective radiation. This variation can be used to direct energy in a particular direction, e.g., perpendicular to the axis, in a forward direction (i.e., towards the distal end of the antenna), or combinations thereof.

Invasive procedures and devices have been developed in which a microwave antenna probe may be either inserted directly into a point of treatment via a normal body orifice or percutaneously inserted. Such invasive procedures and devices potentially provide better temperature control of the tissue being treated. Because of the small difference between the temperature required for denaturing malignant cells and the temperature injurious to healthy cells, a known heating pattern and predictable temperature control is important so that heating is confined to the tissue to be treated. For instance, hyperthermia treatment at the threshold temperature of about 41.5°C generally has little effect on most malignant growth of cells. However, at slightly elevated temperatures above the approximate range of 43°C to 45°C, thermal damage to most types of normal cells is routinely observed. Accordingly, great care must be taken not to exceed these temperatures in healthy tissue.

In the case of tissue ablation, a high radio frequency electrical current in the range of about 300 MHz to about 10 GHz is applied to a targeted tissue site to create an ablation volume, which may have a particular size and shape. Ablation volume is correlated to antenna design, antenna performance, antenna impedance and tissue impedance. The particular type of tissue ablation procedure may dictate a particular ablation volume in order to achieve a desired surgical outcome. By way of example, and without limitation, a spinal ablation procedure may call for a longer, narrower ablation volume, whereas in a prostate ablation procedure, a more spherical ablation volume may be required. In some instances, targeted lesions may be located on or near the surface of the target organ. Such surface lesions have been treated with invasive ablation needles or sticks, which may cause damage to adjacent anatomical structures, increase the likelihood of hemorrhaging, and lengthen operative and recovery times.

WO 2006/084676 A1 discloses a microwave device for the ablation of organic tissues which includes a coaxial antenna insertable in a hollow needle. The antenna has an internal conductor, surrounded by a layer of dielectric material, an external conductor externally coaxial with said layer of dielectric material, a portion of said dielectric material and of said internal conductor protruding from a distal end of said external conductor, to constitute a radiant end of said antenna, a quarter- wave impedance transformer arranged coaxially to said antenna and ending in a short circuit.

US 2007/185554 A1 discloses a minimally-invasive fluid-cooled insertion sleeve device comprising an attached balloon and distally-located penetrating tip.

EP 2 174 614 A1 discloses an apparatus for directing energy to a tissue. The apparatus includes a monopole antenna assembly with a monopole antenna radiating section comprising a monopole antenna element surrounded by a dielectric material.

US 2003/100894 A1 discloses an invasive therapeutic probe system for delivering RF energy to a body tissue. The system includes an RF source configured to provide an RF energy, a probe with a probe shaft and an antenna positioned therein, as well as a feed line for providing conductivity from the RF source to the antenna probe.

WO 02/061880 A2 discloses a microwave antenna for hyperthermia.

### SUMMARY

The invention is defined in claim 1.

The present disclosure is directed to a monopole microwave ablation probe that includes an inner conductor having a first dielectric coaxially disposed thereabout. An outer shield is coaxially disposed around the first dielectric and includes first ground plane electromechanically joined to a distal end thereof. The first ground plane, as well as other ground planes described herein, may be disc-shaped, however, it is to be understood the disclosed ground planes may include any shape, including without limitation ovoid, polygonal, and radial projections. The first ground plane may be oriented substantially transversely to a longitudinal axis of the probe. A distal surface of the ground plane may include a second dielectric disposed thereupon. The inner conductor may extend distally beyond a distal end of the outer shield. The disclosed probe may include a tapered tip joined to a distal end thereof.

In an embodiment, the disclosed ablation probe may further include a second ground plane joined to the outer shield at a juncture proximal of a distal end of the outer shield. The second ground plane may be oriented substantially transversely to a longitudinal axis of the probe. Additionally, a ground plane dielectric may be disposed between the first ground plane and the second ground plane.

A probe in accordance with the present disclosure may include a catheter disposed around a proximal portion of the outer shield to define a conduit therebetween. The conduit may be operably coupled at a distal end thereof to an inflatable balloon disposed around at least the first ground plane. The conduit may be adapted at a proximal end thereof to operably couple to a source of pressure, such as gas pressure or liquid pressure, that may be selectively increased or decreased to inflate and/or deflate the balloon.

Also disclosed is an ablation probe having an inner conductor coaxially disposed within an outer shield, wherein the outer shield defines an inner region around the inner conductor. The probe includes a first ground plane joined to a distal end of the outer shield and oriented substantially transversely to a longitudinal axis of the probe and a second ground plane joined to the outer shield at a juncture proximal of a distal end of the outer shield and oriented substantially transversely to a longitudinal axis of the probe. A generally tubular outer catheter is coaxially disposed around the outer shield to define a cooling conduit between the outer catheter and the outer shield. The disclosed probe may also include a semicylindrical housing having a circumferential surface and a distal surface. A proximal edge of the circumferential surface of the semicylindrical housing is joined to a circumferential edge of the second ground plane. An opening may be defined in the distal surface of the semicylindrical housing, which the inner conductor may extend distally therethough.

Also disclosed is an ablation system having a source of ablation energy operatively coupled to an ablation probe as described herein. The disclosed system may further include at least one of a source of coolant or a source of pressure operatively coupled to an ablation probe as described herein.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is a schematic representation of a microwave ablation system having a surgical ablation probe in accordance with an embodiment of the present disclosure;

Fig. 2 is a perspective view of an embodiment of a microwave ablation probe having a ground plane in accordance with the present disclosure;

Fig. 3 is a side, cutaway view of another embodiment of a microwave ablation probe having a ground plane in accordance with the present disclosure;

Fig. 4 is a side, cutaway view of yet another embodiment of a microwave ablation probe having a ground plane in accordance with the present disclosure;

Fig. 5 is a perspective view of still another embodiment of a microwave ablation probe that includes a ground plane and an inflatable balloon in accordance with the present disclosure;

Fig. 6 is a side, cutaway view of the Fig. 5 embodiment of a microwave ablation probe having a ground plane and an inflatable balloon in accordance with the present disclosure;

Fig. 7 is a perspective view of an embodiment of a microwave ablation probe having a ground plane and a needle electrode in accordance with the present disclosure; and

Fig. 8 is a side, cutaway view of the Fig. 7 embodiment of a microwave ablation probe having a ground plane and a needle electrode in accordance with the present disclosure.

### DETAILED DESCRIPTION

Particular embodiments of the present disclosure are described hereinbelow with reference to the accompanying drawings; however, it is to be understood that the disclosed embodiments are merely examples of the disclosure, which may be embodied in various forms. Well-known and/or repetitive functions and constructions are not described in detail to avoid obscuring the present disclosure in unnecessary or redundant detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In the drawings and in the descriptions that follow, the term "proximal," as is traditional, shall refer to the end of the instrument that is closer to the user, while the term "distal" shall refer to the end that is farther from the user.

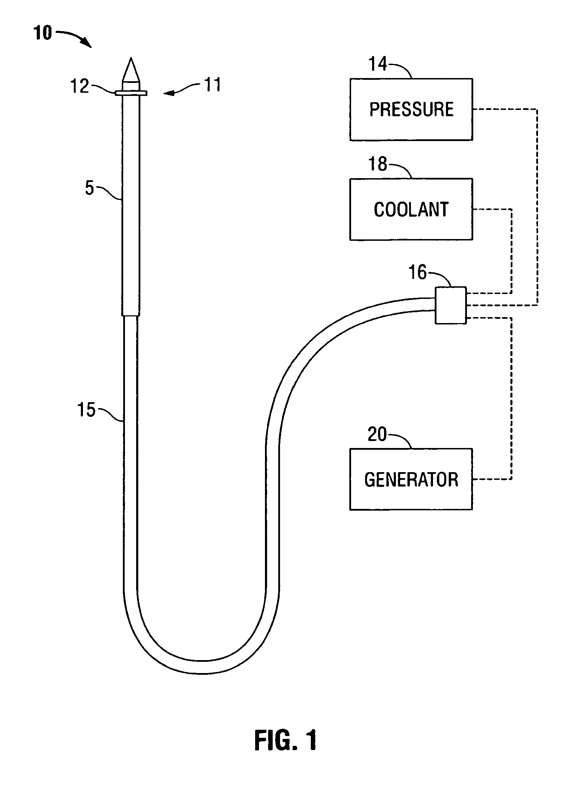

Fig. 1 shows an embodiment of a microwave ablation system 10 in accordance with the present disclosure. The microwave ablation system 10 includes an ablation probe 5 operatively coupled by a cable 15 to connector 16, which may further operably couple the antenna probe 10 to a generator assembly 20. Probe 10 includes a distal radiating portion 11 having a generally circular disc-shaped ground plane 12 disposed thereupon. Generator assembly 20 may be a source of ablation energy, e.g., microwave energy in a range of about 300 MHz to about 10 GHz. In some embodiments, generator assembly 20 may provide ablation energy in a range of about 915 MHz to about 2.45 GHz. Cable 15 may additionally or alternatively provide a conduit (not explicitly shown) configured to provide coolant from a coolant source 18 and/or fluid pressure from a pressure source 14 to the ablation probe 10. Pressure source 14 may be configured to provide pneumatic pressure (e.g., compressed air or other gas) but it is envisioned any suitable pressurized media may be provided by pressure source 14.

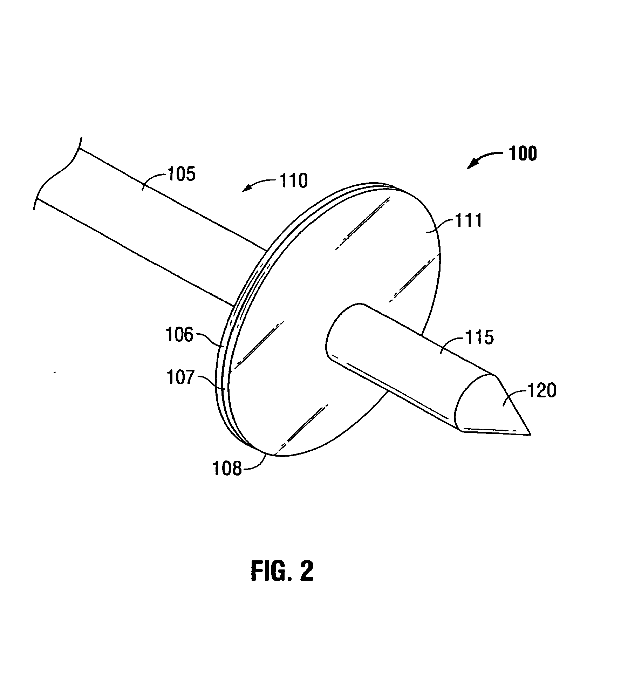

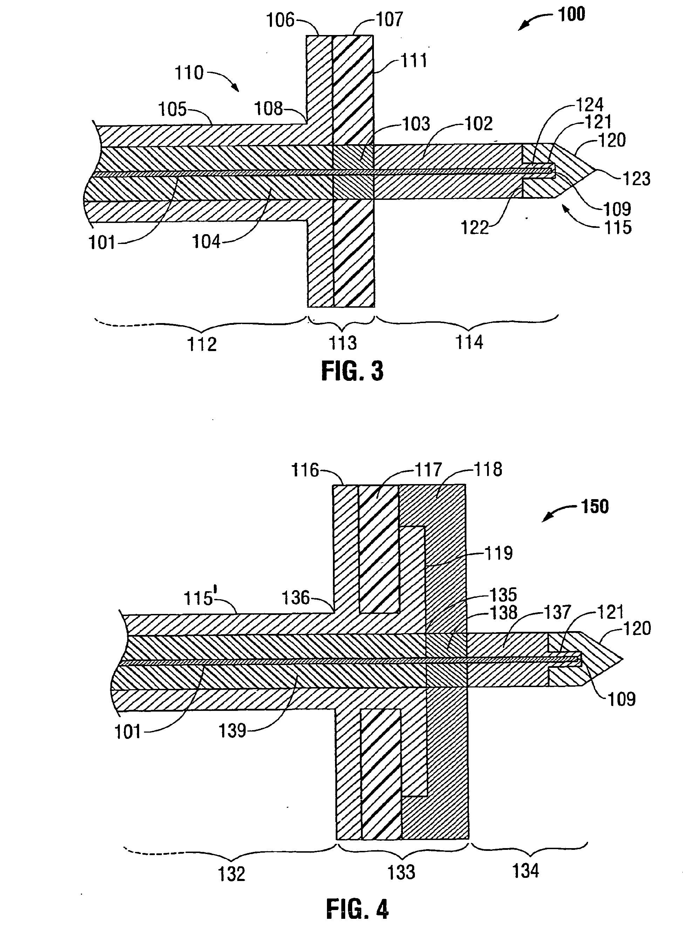

With reference to Figs. 2 and 3, a coaxial fed monopole microwave ablation probe 100 is shown. The disclosed probe 100 includes a shaft assembly 110, a proximal section 112, a ground plane section 113, and a distal section 114. An inner conductor 101 having a distal end 109 is disposed axially though the shaft assembly 110. Inner conductor 101 is coaxially disposed within a proximal dielectric 104, a ground plane inner dielectric 103, and a distal dielectric 102. Proximal dielectric 104, ground plane inner dielectric 103, and/or distal dielectric 102 may be formed from any suitable heat-resistant material having electrically insulative properties, e.g., ceramic, porcelain, or polymeric material. In an embodiment, at least one of proximal dielectric 104, ground plane inner dielectric 103, and/or distal dielectric 102 may be formed from a high-strength dielectric material, e.g., Zirconia or Alumina Zirconia composite.

A distal end 115 of distal dielectric 102 may be joined to a tapered end 120 that terminates at a distal tip 123 to facilitate insertion into tissue with minimal resistance. Alternatively, tip 123 may be rounded or flat. Tapered end 120 includes a proximal base 122 having a recess 121 defined therein to accommodate distal end 109 of inner conductor 101 as shown. A distal portion 124 of distal dielectric 102 may extend into recess 121, which may impart additional strength to a junction between distal dielectric 102 and tapered end 120. Distal dielectric 102 and tapered end 120 may be joined by any suitable manner of attachment, including without limitation adhesive bonding, interference fit, and/or threaded coupling. In an embodiment, distal dielectric section 102 and tapered end 120 may be integrally formed.

Proximal section 112 of probe 100 includes a generally tubular outer shield 105 coaxially disposed about proximal dielectric 104. Outer shield 105 is electromechanically joined at a distal end 108 thereof to a ground plane 106, which has a generally disc-like shape, and is oriented in a substantially transverse configuration with respect to a longitudinal axis of probe 100, e.g., to inner conductor 101. Outer shield 105 and ground plane 106 may be formed from any suitable heat-resistant, electrically-conductive (e.g., metallic) material, including without limitation stainless steel. Outer shield 105 and ground plane 106 may be joined by any suitable manner of attachment, including without limitation, welding, brazing, crimping, soldering, interference fit, and/or threaded coupling. In embodiments, outer shield 105 and ground plane 106 may be integrally formed by, e.g., casting, forging, spin-forming, machining, and the like. Ground plane 106 may include a ground plane surface dielectric 107 disposed on a distal surface thereof. Ground plane surface dielectric 107 may be formed from any suitable heat-resistant material having electrically insulative properties, e.g., ceramic, porcelain, or polymeric material. In some embodiments, proximal section 112, and components thereof (e.g., inner conductor 101, proximal dielectric 104, outer shield 105, etc.) may be formed from substantially rigid materials such that proximal section 112 forms a generally rigid structure. In other embodiments, proximal section 112 and components thereof may be formed from substantially flexible materials such that proximal section 112 forms a generally flexible structure. In this manner, antenna probes in accordance with the present disclosure may be tailored to particular surgical requirements.

In some embodiments, various combinations of proximal dielectric 104, a ground plane inner dielectric 103, ground plane surface dielectric 107, and/or distal dielectric 102 may be integrally formed. Additionally or alternatively, proximal dielectric 104, a ground plane inner dielectric 103, ground plane surface dielectric 107, and distal dielectric 102 may be individually (or in combination) formed from disparate dielectric materials having distinct dielectric properties. In this manner, the electrical properties of probe 100, e.g., impedance, radiation pattern, ablation pattern, etc., may be tailored to surgical requirements.

Additionally or alternatively, an outer surface of probe 100, and/or other embodiments presented herein, e.g., probe 180 and probe 200, discussed below, may include a lubricious, e.g., non-stick coating (not explicitly shown) which may be formed from any suitable heat-resistant conformable material, including without limitation polytetrafluoroethylene (a.k.a. PTFE or Teflon^{®}, manufactured by the E.I. du Pont de Nemours and Co. of Wilmington, Delaware, USA), polyethylene terephthalate (PET), or the like. Additionally or alternatively, an outer surface of the described embodiments may include a heat shrink covering, such as polyolefin tubing, or any suitable heat-shrink material.

With reference to Fig. 4, an embodiment of a monopole microwave ablation probe 150 having a dual ground plane section 133 is shown. Probe 150 includes a proximal section 132 having an outer shield 115' coaxially disposed about proximal dielectric 139. Inner conductor 101 is coaxially disposed within a proximal dielectric 139, a ground plane inner dielectric 138, and a distal dielectric 137. A distal ground plane 119 having a first outer diameter and oriented transversely to inner conductor 101 is electromechanically joined to a distal end 135 of outer shield 115'. A proximal ground plane 116, having a second outer diameter and oriented in spaced relation and substantially parallel to distal ground plane 119, is electromechanically joined to outer shield 115' at a juncture 136 that is located proximally of distal end 135 of outer shield 115'. As seen in Fig. 4, an outer diameter of distal ground plane 119 may be less than an outer diameter of proximal ground plane 116. However, it is to be understood that distal ground plane 119 may have an outer diameter that is greater than, or equal to, an outer diameter of proximal ground plane 116.

A balun dielectric 117 may be disposed between distal ground plane 119 and proximal ground plane 116. Additionally or alternatively, a distal dielectric 118 may be disposed upon at least one of a distal surface of distal ground plane 116 or balun dielectric 117. In use, the described dual ground plane arrangement of distal ground plane 119, proximal ground plane 116, and balun dielectric 117 may facilitate improved control of probe properties, such as without limitation impedance, radiation pattern, and ablation pattern. During use, a proximal end of inner conductor 101 may be operably coupled to a source of ablation energy, e.g., generator 20, to deliver ablation energy to tissue.

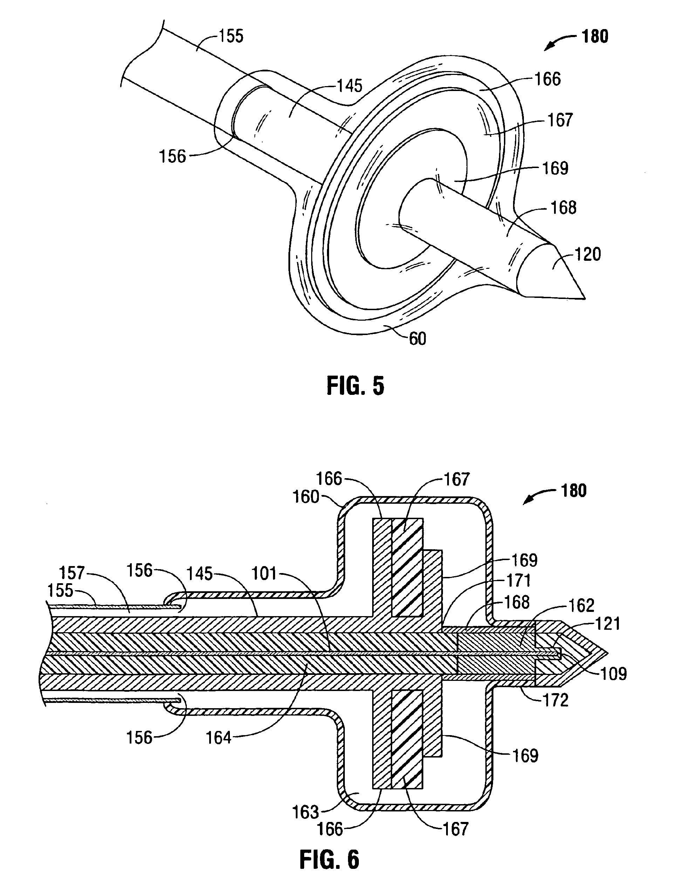

Turning to Figs. 5 and 6, an embodiment of a monopole microwave ablation probe 180 having an inflatable balloon 160 is disclosed. Probe 180 includes an outer shield 145 coaxially disposed about a proximal dielectric 164. Proximal dielectric 164 and distal dielectric 162 are coaxially disposed about inner conductor 101. As can be seen, a distal end 165 of proximal dielectric 164 extends distally of a distal end 171 of outer shield 145. A generally tubular reinforcing member 168 is disposed around the distal end 165 of proximal dielectric 164 and at least a portion of distal dielectric 162. Reinforcing member 168 may be formed from any suitable material, such as without limitation, polyglass composite (e.g., fiberglass), carbon fiber, and the like.

Probe 180 includes a distal ground plane 169 having a first outer diameter and oriented transversely to inner conductor 101. Distal ground plane 169 is electromechanically joined to a distal end 171 of outer shield 145. A proximal ground plane 166 having a second outer diameter and oriented in spaced relation to, and substantially parallel with, distal ground plane 169, is electromechanically joined to outer shield 145 at a juncture 172 that is located proximally of distal end 171 of outer shield 145. An outer diameter of distal ground plane 169 may be less than an outer diameter of proximal ground plane 166, however, it is envisioned that distal ground plane 169 may have an outer diameter that is greater than, or equal to, an outer diameter of proximal ground plane 166. A dielectric 167 may be disposed between distal ground plane 169 and proximal ground plane 166. As shown in Fig. 6, dielectric 167 may have a diameter less than that of proximal ground plane 166 and greater than that of distal ground plane 169; however, dielectric 167 may have a diameter greater than or equal to a diameter of proximal ground plane 166, or, dielectric 167 may have a diameter less than or equal to a diameter of distal ground plane 169.

Inflatable balloon 160 may be disposed around ground planes 166 and 169. Balloon 160 is sealed at a distal end 172 thereof, e.g., to reinforcing member 168, dielectric 162, or tip 120, by any suitable means, including without limitation heat sealing, chemical bonding, adhesive, or mechanical retention (e.g., clamp or ring). A catheter 155 is disposed around a proximal portion of outer shield 145 to define a conduit 157 therebetween. Catheter 155 may be formed from rigid or flexible material. A proximal end 141 of balloon 160 is sealed to a distal end 158 of catheter 155. During use, a proximal end of conduit 157 may be operably coupled to a pressure source 14 to inflate balloon 160. Pressure source 14 may provide gaseous or fluid inflation media, e.g., air, water, saline etc., in a selective manner such that inflation media may be introduced and/or withdrawn from balloon 160 as desired. In an embodiment, pressure source may be manually-operated (bellows, syringe, bulb, etc.) or automated (e.g., a pump). During use, the selective inflation and/or deflation of balloon 160 may enable a surgeon to advantageously alter the electrical properties of probe 180, such as without limitation impedance, radiation pattern, and ablation pattern, as required. Additionally, the selective inflation and/or deflation of balloon 160 may used to control the flow of air, fluids, or other substances into or out of a lumen, during, e.g., endotracheal procedures and the like. Further, the selective inflation and/or deflation of balloon 160 may enable a surgeon to control the positioning of the probe 180 in relation to anatomical structures at or near the operative site.

The balloon 160 may be formed from materials having suitable mechanical properties (such as puncture resistance, pin hole resistance, tensile strength, conformability when inflated), chemical properties (such as forming a suitable bond to the probe 180), and biocompatibility. In one embodiment, the walls of the balloon 160 may be formed from polyurethane having suitable mechanical and chemical properties. One example of a suitable polyurethane is Dow Pellethane® 2363-90A. In another embodiment, the walls of the inflatable balloon 160 may be formed from a suitable polyvinyl chloride (PVC). Other suitable materials include polypropylene, polyethylene teraphthalate (PETP), low-density polyethylene (LDPE), silicone, neoprene, polyisoprene, or polyurethane (PU).

The balloon 160 may be formed in any number of ways, such as without limitation, blow molding, extrusion blow molding, or dip coating. For example, the balloon 160 may be formed from pre-extruded tubing by applying heat and pressure appropriately within a molding cavity to achieve the desired shape (e.g., blow molding). Balloon 160 may also be formed by extrusion blowmolding, wherein melted polymer pellets are extruded through a die to form a tube shape. The still-molten polymer is then captured in a mold, and air pressure is applied to expand the tube to the walls of the mold, thus forming a balloon 160 of the required shape. In yet another example, a substrate (e.g., a form) is immersed into melted polymer. The substrate may be immersed at a constant rate in order to avoid inconsistencies (e.g., "judder"). The substrate is allowed to dwell in the molten polymer for a time sufficient to permit the polymeric material to establish a coating of desired thickness. The substrate is then withdrawn from the molten polymer, preferably, at a constant rate. Additionally or alternatively, the rate at which the substrate is immersed into and/or withdrawn from the molten polymer may affect the thickness of the resultant balloon 160 wall.

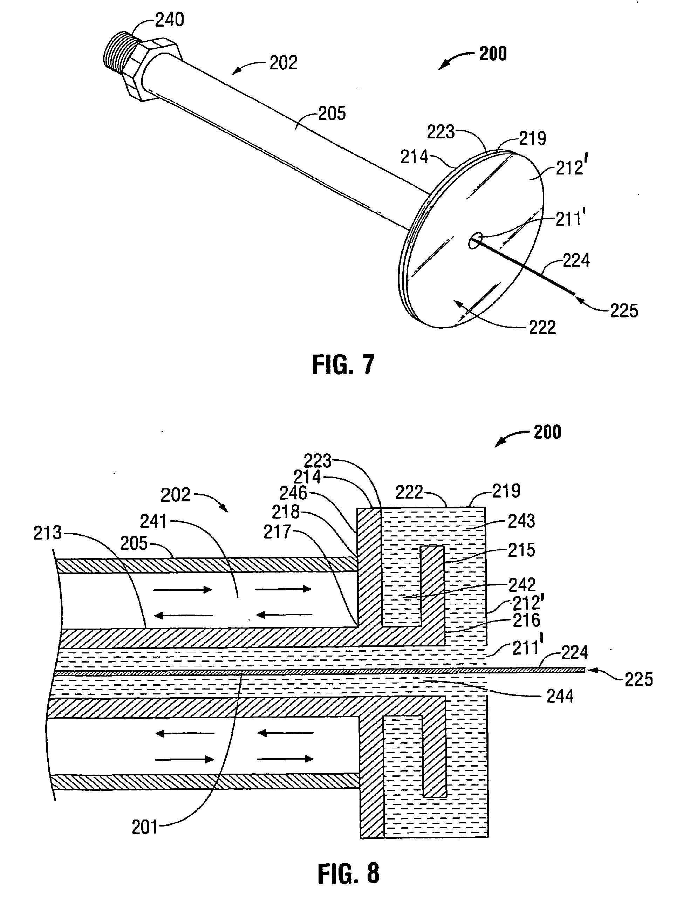

Turning to Figs. 7 and 8, a microwave ablation needle probe 200 having a ground plane 214 is presented. Probe 200 includes a probe shaft 202 having a generally tubular outer catheter 205 coaxially disposed around an outer shield 213 to define a cooling conduit 241 therebetween. Outer catheter 205 may be formed from any suitable fluid-impermeable material, including without limitation, fiberglass composite, carbon fiber, aluminum, stainless steel, and so forth. A generally disc-shaped distal ground plane 215 having a first outer diameter and oriented transversely to inner conductor 201 is fixed to a distal end 216 of outer shield 213. A proximal ground plane 214 having a second outer diameter and oriented in spaced relation to, and substantially parallel with, distal ground plane 215 is electromechanically joined to outer shield 213 at a juncture 217 that is located proximally of distal end 216 of outer shield 213, to form an air gap 242 therebetween. An outer diameter of distal ground plane 215 may be less than an outer diameter of proximal ground plane 214, however, it should be appreciated that distal ground plane 215 may have an outer diameter that is greater than, or equal to, an outer diameter of proximal ground plane 214. A semicylindrical housing 222 having a circumferential surface 291 and a distal surface 212' is joined to a circumferential edge 223 of proximal ground plane 214. An opening 211' is defined at a center of distal surface 212' of semicylindrical housing 222 to accommodate inner conductor 210, as will be described in detail below. An inner volume 243 is defined within semicylindrical housing 222.

A distal end 218 of outer catheter 205 is joined to a proximal surface 246 of proximal ground plane 214 to form a fluid-tight seal. Distal end 218 of outer catheter 205 may be joined to proximal surface 246 of proximal ground plane 214 by any suitable manner of attachment, including adhesive coupling, threaded coupling, welding, soldering, brazing, gasketed coupling, and the like. Alternatively, outer catheter 205 and proximal ground plane 214 may be integrally formed by, e.g., machining, molding, overmolding, and/or forging. Outer shield 213, distal ground plane 215, and/or proximal ground plane 214 may be formed from any suitable heat-resistant, electrically-conductive (e.g., metallic) material, including without limitation stainless steel. Outer shield 213 may be joined to distal ground plane 215 and/or proximal ground plane 214 by any suitable manner of attachment, including without limitation, welding, brazing, crimping, soldering, interference fit, and/or threaded coupling.

An inner conductor 201 is disposed axially through a longitudinal axis of shaft 202 and extends distally through opening 211' and beyond a distal surface 212' of semicylindrical housing 222 to form a needle electrode 224. In an embodiment, needle electrode 224 may include a tip 225 that is sharpened to facilitate the penetration of tissue. Tip 225 may alternatively have a blunt shape, or may include a ball tip (not explicitly shown). Inner conductor 201 may be formed from any suitable biocompatible, electrically conductive material, such as without limitation stainless steel. In an embodiment, needle electrode 224 has sufficient mechanical stiffness to resist or reduce bending during use, e.g., during insertion into, and/or contact with, targeted tissue. Inner conductor 224 is positioned coaxially within an inner region 244 of outer shield 213. A proximal end of inner conductor 201 may be operably coupled to a connector 240 to facilitate a selectively detachable connection between probe 200 and a generator 20 and/or coolant source 18. In embodiments, connector 240 may be an SMA connector, or any other suitable connector adapted to provide an electrical and/or a fluidic coupling.

A proximal end of cooling conduit 241 may be operably coupled to a source of coolant 18. During use, the flow of coolant within cooling conduit 241 may reduce or control temperatures of the probe 200 which, in turn, may enable the delivery of higher power levels to targeted tissue while controlling temperature of non-targeted tissue. Coolant contained within cooling conduit 241 may additionally act as a dielectric. In this manner, improved surgical outcomes, shorter operative times, and reduction of adverse effects to non-targeted tissue may be realized. Additionally, during use, air that is naturally present within air gap 242, inner volume 243 of semicylindrical housing 222, and/or inner region 244 of outer shield 213 may further provide cooling of probe 200 and associated components thereof, e.g., inner conductor 201. It is also envisioned that air and/or other gaseous coolant may be actively or passively circulated within air gap 242, inner volume 243 of semicylindrical housing 222, and/or inner region 244 of outer shield 213 to improve cooling of probe 200.

The described embodiments of the present disclosure are intended to be illustrative rather than restrictive, and are not intended to represent every embodiment of the present disclosure. Further variations of the above-disclosed embodiments and other features and functions, or alternatives thereof, may be made or desirably combined into many other different systems or applications as set forth in the following claims.

## Claims

1. An ablation probe (100; 150; 180), comprising:
an inner conductor (101);
a first dielectric (104; 139; 164) coaxially disposed around the inner conductor (101);
an outer shield (105; 115'; 145) coaxially disposed around the first dielectric (104; 139; 164); and
a first ground plane (106; 119; 169) joined to a distal end (108; 135; 171) of the outer shield (105; 115'; 145) and oriented substantially transversely to a longitudinal axis of the probe (100; 150; 180),
a second dielectric (107; 118; 168) disposed on a distal surface of the first ground plane (106; 119; 169)
**characterized by** a third dielectric (103; 138; 162) disposed between an inner diameter of the second dielectric (107; 118; 168) and the inner conductor (101).

2. The ablation probe according to claim 1, further comprising:
a second ground plane (116; 166) joined to the outer shield (115'; 145) at a juncture (136) proximal of a distal end of the outer shield (115'; 145) and oriented substantially transversely to a longitudinal axis of the probe (150; 180); and
a ground plane dielectric (117; 167) disposed between the first ground plane (119; 169) and the second ground plane (116; 166).

3. The ablation probe according to claim 1,
wherein the inner conductor (101) includes a distal section that extends distally from a distal end of the outer shield (105; 115'; 145).

4. The ablation probe according to claim 3,
further comprising at least a fourth dielectric (102; 137) coaxially disposed around the distal section of the inner conductor (101).

5. The ablation probe according to claim 4,
further comprising a tapered tip (120, 123) joined to a distal end of the fourth dielectric (102; 137).

6. The ablation probe according to claim 3,
wherein the ablation probe further comprises a tapered tip joined to a distal end of the third dielectric (162),
wherein the second dielectric (168) is a reinforcing member disposed around at least a portion of the third dielectric (162), the third dielectric (162) being coaxially disposed around the distal section of the inner conductor (101).

7. The ablation probe according to claim 1,
wherein at least one of the outer shield (105; 115'; 145) or the first ground plane (106; 119; 169) is formed from electrically conductive material.

8. The ablation probe (180) according to claim 1, further comprising:
a catheter (155) disposed around a proximal portion of the outer shield (145) to define a conduit (157) therebetween; and
an inflatable balloon (160) disposed around at least the first ground plane (169).

9. The ablation probe according to claim 8,
wherein the conduit is adapted to operatively couple at a proximal end thereof to a source of pressure.

10. The ablation probe according to claim 8,
wherein the conduit is operatively coupled at a distal end thereof to the inflatable balloon.

11. The ablation probe according to claim 1,
wherein the inner conductor is adapted to operatively couple at a proximal end thereof to a source of ablation energy (20).

## Patentansprüche

1. Ablationssonde (100; 150; 180) mit:
einem Innenleiter (101);
einem um den Innenleiter (101) herum koaxial angeordneten ersten Dielektrikum (104; 139; 164);
einer um das erste Dielektrikum (104; 139; 164) herum koaxial angeordneten Außenabschirmung (105; 115', 145); und
einer ersten Erdungsebene (106; 119; 169), die mit einem distalen Ende (108; 135; 171) der Außenabschirmung (105; 115', 145) verbunden und im Wesentlichen quer zu einer Längsachse der Sonde (100; 150; 180) ausgerichtet ist; und
einem an einer distalen Fläche der ersten Erdungsebene (106; 119; 169) angeordneten zweiten Dielektrikum (107; 118; 168);
**gekennzeichnet durch** ein zwischen einem Innendurchmesser des zweiten Dielektrikums (107; 118; 168) und dem Innenleiter (101) angeordnetes drittes Dielektrikum (103; 138; 162).

2. Ablationssonde nach Anspruch 1, ferner mit:
einer zweiten Erdungsebene (116; 166), die an einer Verbindungsstelle (136) proximal eines distalen Endes der Außenabschirmung (115', 145) mit der Außenabschirmung (115', 145) verbunden und im Wesentlichen quer zu einer Längsachse der Sonde (150; 180) ausgerichtet ist; und
einem zwischen der ersten Erdungsebene (119; 169) und der zweiten Erdungsebene (116; 166) angeordneten Erdungsebenendielektrikum (117; 167).

3. Ablationssonde nach Anspruch 1, bei der der Innenleiter (101) einen sich distal von einem distalen Ende der Außenabschirmung (105, 115', 145) erstreckenden distalen Abschnitt aufweist.

4. Ablationssonde nach Anspruch 3, ferner mit mindestens einem vierten Dielektrikum (102; 137), das koaxial um den distalen Abschnitt des Innenleiters (101) herum angeordnet ist.

5. Ablationssonde nach Anspruch 4, ferner mit einer sich verjüngenden Spitze (120, 123), die mit einem distalen Ende des vierten Dielektrikums (102, 137) verbunden ist.

6. Ablationssonde nach Anspruch 3, bei der
die Ablationssonde ferner eine sich verjüngende Spitze aufweist, die mit einem distalen Ende des dritten Dielektrikums (162) verbunden ist, und
wobei das zweite Dielektrikum (168) ein um mindestens einen Teil des dritten Dielektrikums (162) herum angeordnetes Verstärkungselement ist, wobei das dritte Dielektrikum (162) koaxial um den distalen Abschnitt des Innenleiters (101) herum angeordnet ist.

7. Ablationssonde nach Anspruch 1, bei der die Außenabschirmung (105; 115'; 145) und/oder die erste Erdungsebene (106; 119; 169) aus einem elektrisch leitfähigen Material ausgebildet ist.

8. Ablationssonde (180) nach Anspruch 1, ferner mit:
einem Katheter (155), der derart um einen proximalen Abschnitt der Außenabschirmung (145) herum angeordnet ist, dass dazwischen ein Kanal (157) gebildet ist; und
einem mindestens um die erste Erdungsebene (169) herum angeordneten aufblasbaren Ballon (160).

9. Ablationssonde nach Anspruch 8, bei der der Kanal dazu ausgeführt ist, an einem proximalen Ende desselben mit einer Druckquelle betriebsfähig verbunden zu werden.

10. Ablationssonde nach Anspruch 8, bei der der Kanal an einem distalen Ende desselben mit dem aufblasbaren Ballon betriebsfähig verbunden ist.

11. Ablationssonde nach Anspruch 1, bei der der Innenleiter dazu ausgeführt ist, an einem proximalen Ende desselben mit einer Ablationsenergiequelle (20) betriebsfähig verbunden zu werden.

## Revendications

1. Sonde d'ablation (100 ; 150 ; 180), comprenant :
un conducteur intérieur (101) ;
un premier diélectrique (104 ; 139 ; 164) disposé coaxialement autour du conducteur intérieur (101) ;
une gaine extérieure (105 ; 115' ; 145) disposée coaxialement autour du premier diélectrique (104 ; 139 ; 164) ; et
un premier plan de sol (106 ; 119 ; 169) joint à une extrémité distale (108 ; 135 ; 171) de la gaine extérieure (105 ; 115' ; 145) et orienté sensiblement transversalement à un axe longitudinal de la sonde (100 ; 150 ; 180),
un deuxième diélectrique (107 ; 118 ; 168) disposé sur une surface distale du premier plan de sol (106 ; 119 ; 169)
**caractérisée par** un troisième diélectrique (103 ; 138 ; 162) disposé entre un diamètre intérieur du deuxième diélectrique (107 ; 118 ; 168) et le conducteur intérieur (101).

2. Sonde d'ablation selon la revendication 1, comprenant en outre :
un deuxième plan de sol (116 ; 166) joint à la gaine extérieure (115' ; 145) au niveau d'une jonction (136) proximale d'une extrémité distale de la gaine extérieure (115' ; 145) et orienté sensiblement transversalement à un axe longitudinal de la sonde (150 ; 180) ; et
un diélectrique (117 ; 167) de plan de sol disposé entre le premier plan de sol (119 ; 169) et le deuxième plan de sol (116 ; 166).

3. Sonde d'ablation selon la revendication 1,
dans laquelle le conducteur intérieur (101) inclut une section distale qui s'étend distalement à partir d'une extrémité distale de la gaine extérieure (105 ; 115' ; 145).

4. Sonde d'ablation selon la revendication 3,
comprenant en outre au moins un quatrième diélectrique (102 ; 137) disposé coaxialement autour de la section distale du conducteur intérieur (101).

5. Sonde d'ablation selon la revendication 4,
comprenant en outre une pointe (120, 123) s'effilant jointe à une extrémité distale du quatrième diélectrique (102 ; 137).

6. Sonde d'ablation selon la revendication 3,
dans laquelle la sonde d'ablation comprend en outre une pointe s'effilant jointe à une extrémité distale du troisième diélectrique (162),
dans laquelle le deuxième diélectrique (168) est un élément de renfort disposé autour d'au moins une partie du troisième diélectrique (162), le troisième diélectrique (162) étant disposé coaxialement autour de la section distale du conducteur intérieur (101).

7. Sonde d'ablation selon la revendication 1,
dans laquelle au moins un parmi la gaine extérieure (105 ; 115' ; 145) ou le premier plan de sol (106 ; 119 ; 169) est formé d'un matériau électro-conducteur.

8. Sonde d'ablation (180) selon la revendication 1, comprenant en outre :
un cathéter (155) disposé autour d'une partie proximale de la gaine extérieure (145) pour définir un conduit (157) entre ceux-ci ; et
un ballonnet gonflable (160) disposé autour d'au moins le premier plan de sol (169).

9. Sonde d'ablation selon la revendication 8,
dans laquelle le conduit est adapté à se coupler opérationnellement au niveau d'une extrémité proximale de celui-ci à une source de pression.

10. Sonde d'ablation selon la revendication 8,
dans laquelle le conduit est opérationnellement couplé au niveau d'une extrémité distale de celui-ci au ballonnet gonflable.

11. Sonde d'ablation selon la revendication 1,
dans laquelle le conducteur intérieur est adapté à se coupler opérationnellement au niveau d'une extrémité proximale de celui-ci à une source d'énergie d'ablation (20).
